# EUROPÄISCHE PATENTSCHRIFT

(11) **EP 2 784 200 B1**
(45) Veröffentlichungstag und Bekanntmachung des Hinweises auf die Patenterteilung: **31.01.2018**
(21) Anmeldenummer: 14162183.9
(22) Anmeldetag: 28.03.2014
(51) Int. Cl.: D04C 1/06, D04C 3/48, A61F 2/90

(54) **Verfahren zur Herstellung eines patientenspezifischen Implantats und hergestelltes Implantat**
Method for producing a patient-specific implant and implant made thereby
Procédé de fabrication d'un implant spécifique à un patient et implant obtenu

(30) Priorität: 28.03.2013 DE 102013103176
(43) Veröffentlichungstag der Anmeldung: 01.10.2014
(73) Patentinhaber: Acandis GmbH & Co. KG, 76327 Pfinztal (DE)
(72) Erfinder: Mailänder, Werner, 75331 Engelsbrand Grunbach (DE)
(74) Vertreter: Kilchert, Jochen

(56) Entgegenhaltungen:
- WO-A1-02/086797
- WO-A1-2012/170448
- WO-A2-2010/085794

## Beschreibung

Die Erfindung betrifft ein Verfahren zum Herstellen eines patientenspezifischen, endovaskulären Implantats.

Aus der Praxis sind Implantate, beispielsweise Stents, bekannt, die endovaskulär eingesetzt werden, beispielsweise um eine Gefäßstenose zu behandeln. Derartige Implantate sind meist radial expandierbar, wobei die Implantate eine Radialkraft auf das Gefäß ausüben, um dieses offenzuhalten. Die bisher verfügbaren Implantate werden in Standardgrößen angeboten. Aus einer Vielzahl von standardisierten Größenvarianten wählt der Arzt ein geeignetes Modell aus, um es an einem Patienten einzusetzen.

Die anatomischen Verhältnisse, insbesondere die Formen der Blutgefäße, variieren von Mensch zu Mensch. Insbesondere variiert der Gefäßdurchmesser der Blutgefäße individuell. Die bereitgestellten Implantate weisen jedoch üblicherweise einen konstanten Querschnittsdurchmesser über ihre gesamte Länge auf. Somit ist der behandelnde Arzt gezwungen, ein Implantat einzusetzen, das die anatomischen Verhältnisse des spezifischen Patienten nicht optimal abbildet. Vielmehr wählt der Arzt ein Implantat, das einen Kompromiss zwischen einer ausreichenden Rekanalisationsfunktion und einer geringen Belastung der Gefäßwände bildet. Dabei wird in Kauf genommen, dass an Engstellen des Blutgefäßes eine höhere Radialkraft auf die Gefäßwand wirkt, als dies notwendig ist, um die Gefäßwand an dieser Stelle zu stützen. Es besteht die Gefahr, dass die Gefäßwand stellenweise überreizt wird. Die WO 02/086797 A beschreibt eine Einrichtung zur Darstellung eines virtuellen, den anatomischen Verhältnissen angepassten Katheters. Die WO 2012/170448 A beschreibt eine Einrichtung zur Simulation eines Katheters am Ort der Anwendung. Es ist Aufgabe der vorliegenden Erfindung, ein Verfahren anzugeben, das eine einfache und effiziente Herstellung eines patientenspezifischen Implantats ermöglicht.
Erfindungsgemäß wird diese Aufgabe durch das Verfahren gemäß Anspruch 1 gelöst.
So beruht die Erfindung auf dem Gedanken, ein Verfahren zum Herstellen eines patientenspezifischen, endovaskulären Implantats anzugeben, wobei das Verfahren die folgenden Schritte umfasst:
a. Bereitstellen eines dreidimensionalen, insbesondere virtuellen, Gefäßmodells (11) eines Blutgefäßes;
b. Bildung einer Durchmessersequenz entlang eines Zielabschnitts des Gefäßmodells (11), wobei in regelmäßigen Abständen Durchmesserwerte des Zielabschnitts ermittelt werden;
c. Bildung eines, insbesondere virtuellen, Werkzeugmodells (21) durch Übertragung der Durchmessersequenz auf eine lineare Achse;
d. Erstellung eines Flechtwerkzeugs (31) in der Form des Werkzeugmodells (21); und
e. Bildung des Implantats (41) durch Flechten von Drähten (43) um das Flechtwerkzeug (31).
Die vorgenannten Verfahrensschritte werden vorzugsweise in der angegebenen Reihenfolge durchgeführt. Mit dem erfindungsgemäßen Verfahren ist es möglich, auf einfache und effiziente, insbesondere schnelle, Art und Weise ein Implantat herzustellen, das den anatomischen Gegebenheiten eines spezifischen Patienten Rechnung trägt. Als Basis für das Herstellungsverfahren dient das dreidimensionale Gefäßmodell, das auch virtuell vorliegen kann. In einer bevorzugten Ausführungsform kann das Gefäßmodel Bilderfassungsdaten aufweisen. Die Bilderfassungsdaten können beispielsweise durch ein angiographisches Bilderfassungssystem bereitgestellt werden. Konkret kann anhand von dreidimensionalen, angiographischen Patientendaten bzw. Bilderfassungsdaten das Gefäßmodell, zumindest virtuell, erstellt werden. Das dreidimensionale Gefäßmodell umfasst vorzugsweise Durchmesserdaten, Krümmungsdaten und/oder Längendaten des Blutgefäßes.

In einem nächsten Schritt wird entlang eines Zielabschnitts des Geflechtmodells, beispielsweise im Bereich eines Aneurysmas oder einer Stenose, eine Durchmessersequenz ermittelt. Konkret kann sequentiell der Gefäßdurchmesser des Gefäßmodells bzw. des Blutgefäßes entlang des Zielabschnitts vermessen werden. Anschließend kann aus den gemessenen Durchmesserwerten, insbesondere der Durchmessersequenz, die einerseits einzelne Durchmesserwerte und andererseits Längenabstände bzw. Positionsdaten der einzelnen Durchmesserwerte umfasst, ein Werkzeugmodell erstellt werden. Dabei wird die Durchmessersequenz auf eine lineare Achse übertragen bzw. projiziert. Mit anderen Worten wird das dreidimensionale Gefäßmodell, das Gefäßkrümmungen darstellen kann, zumindest virtuell gestreckt, so dass die Mittelachse des Gefäßmodells, das ein Blutgefäß repräsentiert, entlang einer Geraden verläuft. Dabei kann das Werkzeugmodell rotationssymmetrisch ausgebildet sein.

Das Werkzeugmodell kann als virtuelles Modell vorliegen. Mit anderen Worten kann das Werkzeugmodell Modelldaten aufweisen, die beispielsweise an ein CAD-System übertragbar sind. Insbesondere kann das Werkzeugmodell als Datensatz im STL-Format oder im IGES-Format bereitgestellt werden.

Anhand des Werkzeugmodells wird in einem nächsten Verfahrensschritt ein Flechtwerkzeug hergestellt. Das Flechtwerkzeug weist dabei die Form des Werkzeugmodells auf. Beispielsweise kann der Datensatz eines virtuellen Werkzeugmodells unmittelbar an eine CAD/CAM-Schnittstelle übergeben werden, so dass das Flechtwerkzeug unmittelbar auf Grundlage des Datensatzes des Werkzeugmodells hergestellt werden kann.

Das Flechtwerkzeug dient vorzugsweise als Kern zum Flechten eines Implantats. Beim Flechten des Implantats können ein oder mehrere Drähte im Wesentlichen helixförmig um das Flechtwerkzeug gewunden werden. Auf diese Weise kann ein Drahtgeflecht gebildet werden, das die Außenkontur des Flechtwerkzeugs, d.h. indirekt die Außenkontur des Werkzeugmodells, übernimmt. Das so hergestellte Implantat ist also an die Anatomie eines spezifischen Patienten angepasst. Im Gebrauch stützt das Implantat so ein Gefäß, ohne eine punktuell übermäßige Radialkraft auf die Gefäßwand aufzubringen. Vielmehr kann das Implantat derart gestaltet werden, dass die Radialkraft über die gesamte Implantatlänge im Blutgefäß gleich ist. Dies schont die Gefäßwände und ermöglicht dennoch eine effiziente Rekanalisation des Blutgefäßes.

In einer weiteren bevorzugten Ausführungsform der Erfindung kann vorgesehen sein, dass bei der Bildung des Werkzeugmodells dessen Außenkontur geglättet und/oder dessen axiale Enden modifiziert werden. Beispielsweise kann das Werkzeugmodell in eine rotationssymmetrische Geometrie überführt werden, indem die Außenkontur des Werkzeugmodells geglättet wird. Ferner kann vorgesehen sein, die Implantat-Enden durch Anpassung des Werkzeugmodells zu gestalten, beispielsweise die Implantat-Enden radial nach außen aufweitend zu konstruieren. So kann an den axialen Enden des Implantats eine verbesserte Verankerungsfunktion bereitgestellt werden.

Besonders bevorzugt ist es, wenn die Schritte des Bereitstellens des Gefäßmodells, der Bildung der Durchmessersequenz und/oder der Bildung des Werkzeugmodells durch ein Bildbearbeitungsprogramm auf einem Rechner ausgeführt werden. Dies stellt eine besonders einfache Handhabung der einzelnen Modelle dar. Insbesondere können das Gefäßmodell und das Werkzeugmodell durch Datensätze gebildet werden, die durch das Bildbearbeitungsprogramm auf einem Display darstellbar sind. Das Bildbearbeitungsprogramm kann Schnittstellen zu weiteren Programmen, beispielsweise eine Werkzeugmodelldatenschnittstelle zur Übergabe der Werkzeugmodelldaten an ein CAD/CAM-System aufweisen. Konkret ist bei dem Herstellungsverfahren in wenigstens einer bevorzugten Ausführungsform vorgesehen, dass beim Flechten der Drähte eine, insbesondere rotationssymmetrische, Umfangswandung mit einer Außenkontur gebildet wird, die der Innenkontur des Blutgefäßes bzw. des Zielabschnitts entspricht. Mit anderen Worten kann das Implantat eine zur Form bzw. Innenkontur des Blutgefäßes bzw. des Zielgefäßes komplementäre Form aufweisen.

Grundsätzlich kann vorgesehen sein, dass das endovaskuläre Implantat radial komprimierbar und/oder expandierbar ist. Das endovaskuläre Implantat ist vorzugsweise aus einem Geflecht von Drähten gebildet, das zur Zuführung des Implantats in das Blutgefäß radial komprimierbar ist. Vorzugsweise ist das Implantat selbstexpandierbar, so dass sich das Implantat im Zielabschnitt bzw. im Blutgefäß selbsttätig radial aufweitet, sobald es aus einem Zuführsystem entlassen wird. Das endovaskuläre Implantat kann ein Stent sein.

Bei einer weiteren Ausführungsform des erfindungsgemäßen Verfahrens ist vorgesehen, dass beim Flechten der Drähte ein Flechtwinkel zwischen zwei sich kreuzenden Drähten variiert wird. Die Variation des Flechtwinkels erfolgt vorzugsweise derart, dass in Längsrichtung des Implantats zumindest abschnittsweise ein konstanter Maschenwinkel gebildet wird. Dies gilt für den Ruhezustand des Implantats, also den kraftunbelasteten Zustand des Implantats. Beim Flechten der Drähte kann auch eine Abzugsgeschwindigkeit der Drähte variiert werden, so dass in Längsrichtung des Implantats zumindest abschnittsweise ein konstanter Maschenwinkel gebildet wird. Der konstante Maschenwinkel wird vorzugsweise vollständig über ein Abschnitt des Implantats gebildet, der unterschiedliche Querschnittsdurchmesser aufweist.

Es hat sich gezeigt, dass ein Implantat, welches im Ruhezustand unabhängig von der Außenkontur, also unabhängig von einer Querschnittsdurchmesseränderung, zumindest abschnittsweise einen konstanten Maschenwinkel aufweist, eine höhere Flexibilität bereitstellt. Ein derartiges Implantat, beispielsweise ein Stent aus einem Drahtgeflecht, kann sich gut an Krümmungen in Blutgefäßen anpassen und eine unerwünschte Beeinflussung des Blutstroms vermeiden. Um sicherzustellen, dass der Maschenwinkel am fertiggestellten Implantat über zumindest einen Abschnitt des Implantats, insbesondere über das vollständige Implantat konstant ist, wird der Flechtwinkel oder die Abzugsgeschwindigkeit der Drähte vorzugsweise variiert, um die Durchmesseränderungen in der Außenkontur des Implantats auszugleichen. Als Maschenwinkel wird im Rahmen der Anmeldung grundsätzlich ein Winkel verstanden, der sich zwischen zwei sich kreuzenden Drähten einer Masche zeigt. Insbesondere werden diejenigen Winkel zwischen zwei sich kreuzenden Drähten als Maschenwinkel bezeichnet, die eine in Längsrichtung bzw. Axialrichtung des Implantats ausgerichtete Winkelhalbierende aufweisen.

Die Größe des Maschenwinkels wird durch den eingestellten Flechtwinkel beim Flechten des Implantats bzw. Geflechts bestimmt. Der Flechtwinkel ist der spitze Winkel, den ein Draht beim Flechten bzw. Wickeln um das Flechtwerkzeug mit der Längsachse des Flechtwerkzeugs einschließt. Vorzugsweise werden für Drähte die in unterschiedlichen Spiralrichtungen um das Flechtwerkzeug bzw. den Flechtboden gewickelt werden, gleiche Flechtwinkel eingestellt. So ist gewährleistet, dass die Winkelhalbierende des resultierenden Maschenwinkels parallel zur Längsachse des Implantats bzw. Geflechtsröhrchens ausgerichtet ist. Als Abzugsgeschwindigkeit wird die Geschwindigkeit bezeichnet, mit welcher der Draht von einer Spule in der Flechtmaschine abgewickelt bzw. mit welcher der Draht um den Flechtdorn gewickelt wird. Mit der Erfindung kann ein endovaskuläres Implantat hergestellt werden, das abschnittsweise unterschiedliche Querschnittsdurchmesser und gleichzeitig eine hohe Biegeflexibilität aufweist.

Dieser nebengeordnete Aspekt der Erfindung beruht auf dem Gedanken, ein endovaskuläres Implantat, insbesondere einen Stent, mit einem rohrförmigen Geflecht aus Drähten anzugeben, die Maschen bilden und sich in jeder Masche unter einem Maschenwinkel kreuzen. Das Geflecht weist zumindest einen Abschnitt mit unterschiedlichen Querschnittsdurchmessern auf. Wie aus der WO 2010/085794 A2 bekannt, ist der Maschenwinkel zumindest entlang des Abschnitts des Geflechts konstant.
Im Allgemeinen ist vorgesehen, dass sich der konstante Maschenwinkel in einem Ruhezustand des Geflechts zeigt, d.h. in einem Zustand, in welchem das Geflecht keinen äußeren Kräften ausgesetzt ist. Das Geflecht weist im Ruhezustand insbesondere eine rohrförmige Außenkontur mit unterschiedlichen Querschnittsdurchmessern auf. Der konstante Maschenwinkel stellt sicher, dass die Flexibilitätseigenschaften, insbesondere hinsichtlich der Biegeflexibilität, des Implantats, zumindest über den Abschnitt des Geflechts unabhängig von lokalen Änderungen des Querschnittsdurchmessers gleich ist.

Bei dem Geflecht kann zusätzlich vorgesehen sein, dass in Umfangsrichtung des Implantats unmittelbar benachbarte Maschen einen Maschenring bilden, der einen einheitlichen Maschenwinkel aufweist. Im Wesentlichen ist also vorzugsweise vorgesehen, dass nicht nur in Längsrichtung benachbarte Maschen durch Drähte gebildet sind, die einen konstanten Maschenwinkel aufweisen, sondern dass dies auch für in Umfangsrichtung des Geflechts unmittelbar benachbarte Maschen gilt. Der Abschnitt des Geflechts, der einen einheitlichen Maschenwinkel aufweist, umfasst also einen rohrförmigen Teilabschnitt des Geflechts, im welchem alle sich kreuzenden Drähte den einheitlichen Maschenwinkel bilden. Es ist auch möglich, dass das gesamte rohrförmige Geflecht, insbesondere das gesamte Implantat einen einheitlichen, konstanten Maschenwinkel aufweist.

Alternativ kann vorgesehen sein, dass der Maschenwinkel entlang des Geflechts variiert wird. Insbesondere kann der Maschenwinkel in Längsrichtung des Geflechts variiert werden. Dabei können in Umfangsrichtung benachbarte Maschen, die vorzugsweise einen Maschenring bilden, jeweils gleiche Maschenwinkel aufweisen. Es ist auch möglich, dass die Maschen eines Maschenrings unterschiedliche Maschenwinkel aufweisen.

Insofern wird im Rahmen der vorliegenden Anmeldung auch ein endovaskuläres Implantat, insbesondere ein Stent, offenbart, der ein rohrförmiges Geflecht aus Drähten aufweist, die Maschen bilden. Die Drähte kreuzen sich in jeder Masche unter einem Maschenwinkel, wobei das Geflecht zumindest einen Abschnitt mit unterschiedlichen Querschnittsdurchmessern aufweist. Dabei kann der Maschenwinkel so entlang des Geflechts variieren, dass die Porosität des gesamten Geflechts einheitlich ist. Unter Porosität wird im Rahmen der vorliegenden Anmeldung das Verhältnis zwischen offener bzw. durchlässiger Fläche und Gesamtfläche des Geflechts verstanden. Die offene bzw. durchlässige Fläche des Geflechts wird durch die Maschen abzüglich der Drahtdurchmesser gebildet. Die Porosität kann auch so beschrieben werden, dass in jede Masche ein Kreis einbeschrieben werden kann, der wenigstens drei die Masche begrenzende Drähte berührt. Vorzugsweise ist ein solcher Maschen-Inkreis über alle Maschen des Geflechts konstant.

In diesem Zusammenhang wird darauf hingewiesen, dass die hier beschriebenen konstanten oder variierenden Maschenwinkel sowie die hier beschriebene, konstante Porosität im expandierten Zustand des Geflechts vorliegen. Maßgeblich ist also die Gestaltung des Implantats im expandierten Zustand, d.h. im Ruhezustand, wobei das Implantat keinen äußeren Kräften ausgesetzt ist.

Um die gleichmäßige Porosität zu erreichen, kann beispielsweise in Abschnitten des Geflechts, die einen kleineren Querschnittsdurchmesser als benachbarte Abschnitte aufweisen, der Maschenwinkel reduziert werden, falls der Maschenwinkel in den benachbarten Abschnitten größer als 45° ist. Mit anderen Worten führt eine Verkleinerung des Maschenwinkels in einem Winkelbereich oberhalb von 45° dazu, dass eine Durchmesser-Reduktion ausgeglichen wird und die Porosität insofern konstant bleibt. Der Grad der Verkleinerung kann anhand von trigonometrischen Grundfunktionen einfach durch den Fachmann errechnet und eingestellt werden.

Die Erfindung wird im Folgenden anhand eines Ausführungsbeispiels unter Bezugnahme auf die beigefügten, schematischen Zeichnungen näher erläutert. Darin zeigen
- Fig. 1: eine schematische Darstellung der Abfolge der einzelnen Verfahrensschritte des erfindungsgemäßen Verfahrens gemäß einem bevorzugten Ausführungsbeispiel;
- Fig. 2: eine Darstellung des dreidimensionalen Gefäßmodells eines Blutgefäßes;
- Fig. 3: eine Darstellung eines Werkzeugmodells, das anhand einer Durchmessersequenz des Gefäßmodells gemäß Fig. 2 erstellt ist;
- Fig. 4: eine Seitenansicht eines Flechtwerkzeugs, das anhand des Werkzeugmodells gemäß Fig. 3 hergestellt ist;
- Fig. 5: eine Seitenansicht eines Implantats, das mit dem erfindungsgemäßen Verfahren hergestellt ist, insbesondere anhand des Werkzeugmodells gemäß Fig. 4;
- Fig. 6: eine Seitenansicht eines erfindungsgemäßen Implantats nach einem bevorzugten Ausführungsbeispiel, wobei das Geflecht des Implantats über die gesamte Implantatlänge einen konstanten Maschenwinkel aufweist;
- Fig. 7: eine Seitenansicht des Implantats gemäß Fig. 5, wobei zusätzlich einzelne Abschnitte mit jeweils konstantem Maschenwinkel schematisch dargestellt sind.

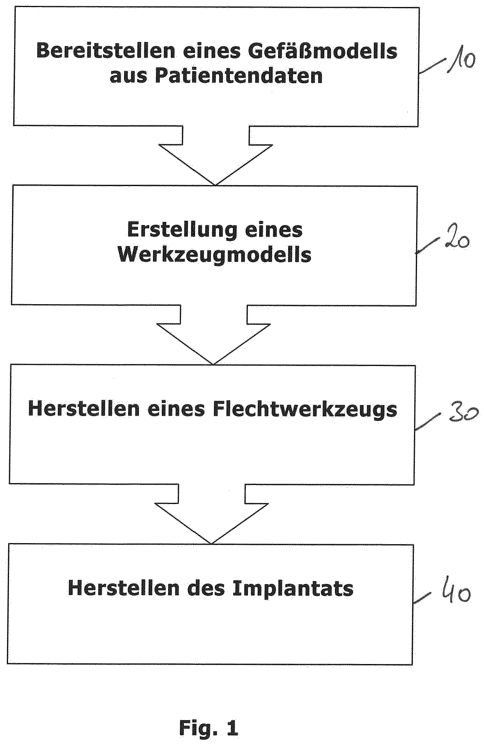

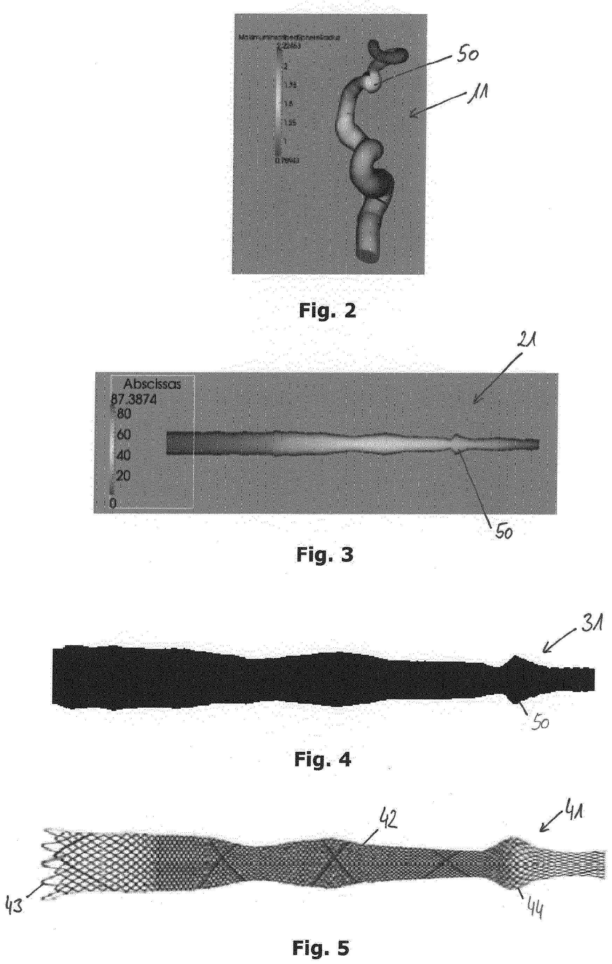

Fig. 1 zeigt in schematischer Weise die Schrittabfolge des erfindungsgemäßen Verfahrens. Bei dem erfindungsgemäßen Verfahren wird in einem ersten Schritt 10 ein dreidimensionales, vorzugsweise virtuelles, Gefäßmodell 11 eines Blutgefäßes bereitgestellt. Insbesondere wird das Gefäßmodell 11 aus Patientendaten, vorzugsweise aus einem dreidimensionalen angiographischen Patientendatensatz, generiert. Das Gefäßmodell 11 kann graphisch auf einem Display eines Rechners dargestellt werden. Eine derartige Darstellung ist beispielhaft in Fig. 2 gezeigt. Auf Einzelheiten der Darstellung gemäß Fig. 2 wird später näher eingegangen.

In einem zweiten Schritt 20 wird ein Werkzeugmodell 21 erstellt. Die Erstellung des Werkzeugmodells 21 erfolgt vorzugsweise virtuell, insbesondere durch ein Bildbearbeitungsprogramm. Zur Erstellung des Werkzeugmodells 21 wird zunächst entlang des Gefäßmodells 11 ein Zielabschnitt definiert. Der Zielabschnitt entspricht einem Behandlungsabschnitt des Blutgefäßes, der später mit dem Implantat zu behandeln ist. Im Zielabschnitt des Gefäßmodells 11 wird in regelmäßigen Abständen entlang der Mittelachse des Gefäßmodells 11 ein Durchmesserwert erfasst. So wird eine Vielzahl von diskreten Durchmesserwerten zu einer Durchmessersequenz zusammengefasst. Die Durchmessersequenz repräsentiert den Zielabschnitt, wobei jedem einzelnen Durchmesserwert eine diskrete Position entlang des Zielabschnitts zugewiesen ist. Anschließend wird das Werkzeugmodell 21 gebildet, indem die Durchmessersequenz auf eine lineare Achse projiziert bzw. übertragen wird. Die einzelnen, diskreten Durchmesserwerte werden also entlang einer linearen Achse aufgetragen, so dass sich das virtuelle Werkzeugmodell 21 ergibt. Das Werkzeugmodell 21 entspricht im Wesentlichen einer linear gestreckten Projektion des Zielabschnitts. Fig. 3 zeigt ein Werkzeugmodell 21, das anhand des Gefäßmodells 11 gemäß Fig. 2 gebildet ist.

Das Werkzeugmodell 21 dient in einem dritten Schritt 30 des Herstellungsverfahrens als Vorlage für die Herstellung eines Flechtwerkzeugs 31, insbesondere eines Flechtdorns. Anhand der Werkzeugmodelldaten kann insbesondere eine Werkzeugmaschine derart programmiert bzw. gesteuert werden, dass ein Flechtwerkzeug 31, insbesondere ein Flechtdorn, mit der dem Werkzeugmodell entsprechenden Außenkontur hergestellt wird.

Das Werkzeugmodell 21 kann vor dem dritten Schritt 30 modifiziert werden. Beispielsweise kann das Werkzeugmodell 21 geglättet werden, so dass sich insgesamt ein rotationssymmetrisches Werkzeugmodell 21 ergibt. Insbesondere können die Übergänge zwischen den diskreten Durchmesserwerten geglättet werden. Fig. 3 zeigt ein geglättetes Werkzeugmodell 21. Ferner kann vorgesehen sein, das Werkzeugmodell 21 hinsichtlich der axialen Enden 22 anzupassen, so dass das Werkzeugmodell 21 die Herstellung von modifizierten Implantat-Enden ermöglicht. Vorzugsweise wird das Flechtwerkzeug 31 im dritten Schritt 30 anhand des modifizierten Werkzeugmodells 21 gefertigt.

In einem letzten, vierten Schritt 40 des Verfahrens wird das Implantat 41 hergestellt, indem ein oder mehrere Drähte 43 um das Flechtwerkzeug 31 geflochten werden. Insbesondere wird auf diese Weise ein Gittergeflecht bzw. ein Drahtgeflecht hergestellt, das im Wesentlichen rohrförmig ausgebildet ist. Dabei weist das Drahtgeflecht eine Kontur, insbesondere Außenkontur, auf, die der Außenkontur des Flechtwerkzeugs 31 bzw. des Werkzeugmodells 21 entspricht. Fig. 5 zeigt ein Implantat 41, das auf dem Flechtwerkzeug 31 bzw. Flechtdorn gemäß Fig. 4 hergestellt wurde.

In Fig. 2 ist ein Gefäßmodell 11 eines Blutgefäßes dargestellt. Das Gefäßmodell 11 kann aus angiographischen Daten erstellt sein, die von einem spezifischen Patienten stammen. Wie in Fig. 2 gut erkennbar ist, weist das Gefäßmodell, analog zum realen Blutgefäß, Krümmungen und unterschiedliche Gefäßdurchmesser auf. Insbesondere ist im Gefäßmodell 11 eine Aneurysmenprojektion 50, d.h. die virtuelle Wiedergabe der Form eines Aneurysmas, erkennbar, das als lokale Ausweitung des Gefäßmodells dargestellt ist. Zur Abschirmung der Aneurysmenprojektion 50 vom Blutstrom, kann beispielsweise ein Implantat eingesetzt werden, das nach dem erfindungsgemäßen Verfahren hergestellt ist. Dazu wird zunächst ein Zielabschnitt des Gefäßmodells 11 festgelegt. Der Zielabschnitt wird anschließend vermessen, so dass diskrete Durchmesserwerte in Form einer Durchmessersequenz vorliegen. Die Durchmessersequenz wird auf eine lineare Achse übertragen, um das Werkzeugmodell 21 bereitzustellen.

Fig. 3 zeigt das Werkzeugmodell 21, das anhand des Gefäßmodells 11 gemäß Fig. 2 erstellt bzw. berechnet ist. Bei dem Werkzeugmodell 21 gemäß Fig. 3 ist insbesondere der Bereich der Aneurysmenprojektion 50 gut erkennbar. An der Stelle der Aneurysmenprojektion 50 weist das Werkzeugmodell 21 eine prägnante Ausweitung auf.
Fig. 4 zeigt das anhand des Werkzeugmodells 21 hergestellte Flechtwerkzeug 31 in einer Seitenansicht. Es ist gut erkennbar, dass die Außenkontur des Flechtwerkzeugs 31 im Wesentlichen der Außenkontur des Werkzeugmodells 21 entspricht. Insbesondere ist der Bereich der Aneurysmenprojektion 50 bei dem Flechtwerkzeug 31 gut und prägnant abgebildet.
In Fig. 5 ist ein Implantat 41, insbesondere ein Stent, gezeigt, das an die Anatomie des Zielabschnitts des Gefäßmodells 11 gemäß Fig. 2 und somit an die Anatomie des spezifischen Patienten, anhand dessen das Gefäßmodell 11 erstellt ist, angepasst ist. Insbesondere ist gut erkennbar, dass die Außenkontur des Implantats 41 im Wesentlichen der Außenkontur des Flechtwerkzeugs 31 bzw. des Werkzeugmodells 21 entspricht. Das Implantat 41 weist eine Umfangswandung 42 auf, die durch miteinander verflochtene Drähte 43 gebildet ist. Die Umfangswandung 42 verläuft im Wesentlichen rotationssymmetrisch um eine Längsachse, insbesondere eine lineare Längsachse, des Implantats 41. Insbesondere weist die Umfangswandung 42 bzw. das Implantat 41 unterschiedliche Querschnittsdurchmesser auf, wobei die Variation der Querschnittsdurchmesser der Durchmessersequenz entspricht, die Vorlage für die Bildung des Werkzeugmodells 21 ist. Insofern bildet das Implantat 41 die Anatomie des Zielabschnitts des Gefäßmodells bzw. des Blutgefäßes des Patienten nahezu identisch ab. Das Implantat 41, insbesondere der Stent, umfasst konkret eine Aneurysmenausweitung, die im Wesentlichen der Durchmessererweiterung im Bereich der Aneurysmenprojektion 50 entspricht. Die Aneurysmenausweitung 44 ist im implantierten Zustand des Stents auf Höhe des Aneurysmas angeordnet, welches die Form der Aneurysmenprojektion vorgegeben hat, und deckt das Aneurysma insofern ab.

Grundsätzlich ist das herstellte Implantat 41 für die Behandlung von Aneurysmen in Blutgefäßen, insbesondere in zerebralen Blutgefäßen, geeignet. Des Weiteren kann das Implantat 41 bzw. der Stent zur Behandlung von Stenosen in Blutgefäßen eingesetzt werden. Grundsätzlich ist die Erfindung nicht auf den zerebralen Anwendungsbereich beschränkt. Generell kann das mit dem erfindungsgemäßen Verfahren hergestellte Implantat 41 auch bei peripheren Blutgefäßen, kardiovaskulär und/oder neurovaskulär eingesetzt werden.

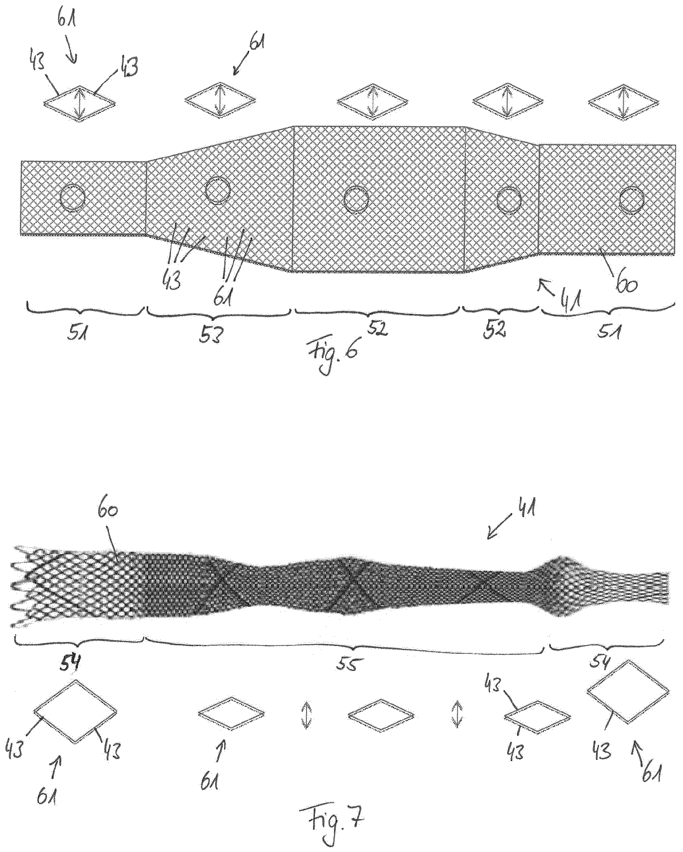

Die Fig. 6 und 7 zeigen beispielhaft jeweils ein endovaskuläres Implantat 41, das nach dem erfindungsgemäßen Verfahren hergestellt ist. Die beiden Implantate 41 gemäß Fig. 6 und 7 weisen ein rohrförmiges Geflecht 60 aus Drähten 43 auf, wobei das Geflecht 60 jeweils wenigstens einen Abschnitt mit unterschiedlichen Querschnittsdurchmessern umfasst. Konkret ist bei den Implantaten 41 gemäß Fig. 6 und 7 vorgesehen, dass sich der Querschnittsdurchmesser des Geflechts in Längsrichtung des Geflechts 60 ändert. Die Änderung des Querschnittsdurchmessers in Längsrichtung des Geflechts 60 ist vorzugsweise der Patientenanatomie geschuldet, so dass es sich um patientenspezifische Implantate 41 handelt. Beide in den Fig. 6 und 7 dargestellte Implantate 41 weisen Drähte 43 auf, die zumindest abschnittsweise entlang des rohrförmigen Geflechts 60 einen einheitlichen Maschenwinkel aufweisen.

Der Maschenwinkel der Drähte 43 des Implantats 41 ist als der Winkel definiert, der sich innerhalb einer Masche 61 zwischen zwei Drähten 43 zeigt, die in unterschiedlichen Spiralrichtungen um die Längsachse des Geflechts 60 gewunden sind. Dabei wird der spitze Winkel zwischen den Drähten 43 betrachtet. Der Maschenwinkel ist also größer als 0° und kleiner als 180°.

Bei den Ausführungsbeispielen gemäß Fig. 6 weisen die Drähte 43 des gesamten rohrförmigen Geflechts 60 einen einheitlichen Maschenwinkel auf. Der Maschenwinkel ist also über die gesamte Länge des rohrförmigen Geflechts 60 konstant. Dies wird bei der Herstellung des Implantats 41 dadurch erreicht, dass der Flechtwinkel bereichsweise variiert wird, um die Durchmesseränderungen des Geflechts 60 auszugleichen. In Fig. 6 ist gut erkennbar, dass das Geflecht 60 mehrere in Längsrichtung benachbarte Bereiche 51, 52, 53 aufweist, die unterschiedliche Querschnittsdurchmesser umfassen. Insbesondere sind zwei Randbereiche 51 vorgesehen, die voneinander verschiedene Querschnittsdurchmesser aufweisen, wobei beide Randbereiche 51 eine zylinderförmige Außenkontur bilden. Ferner ist ein Mittelbereich 52 vorgesehen, der einen Querschnittsdurchmesser aufweist, welcher größer als der Querschnittsdurchmesser der Randbereiche 51 ist. Der Mittelbereich 52 bildet ebenfalls eine zylinderförmige Außenkontur. Zwischen dem Mittelbereich 52 und jeweils einem Randbereich 51 erstreckt sich ein Übergangsbereich 53. Insgesamt weist das Geflecht 60 bzw. Implantat 41 gemäß Fig. 6 zwei Übergangsbereiche 53 auf, die eine kegelstumpfförmige Außenkontur aufweisen.

Bei der Herstellung des Implantats 41 wird das Geflecht 60 durch Umwickeln des Flechtdorns bzw. Flechtwerkzeugs 31 mit den Drähten 43 gebildet. Dabei werden entlang der einzelnen Bereiche, insbesondere der Randbereiche 51, des Mittelbereichs 52 und der Übergangsbereiche 53, der Flechtwinkel und/oder die Abzugsgeschwindigkeit der Drähte 43 variiert, so dass sich im fertiggestellten Zustand des Geflechts 60 bzw. Implantats 41 ein über die gesamte Länge des Implantats einheitlicher, konstanter Maschenwinkel einstellt. Der konstante Maschenwinkel ist insbesondere im Ruhezustand des Geflechts 60 erkennbar.

Es ist auch möglich, dass das Implantat mehrere Abschnitte 54, 55 aufweist, die unterschiedliche Maschenwinkel umfassen. Dabei umfasst wenigstens ein Abschnitt 54, 55 einen konstanten Maschenwinkel. Ein derartiges Ausführungsbeispiel ist in Fig. 7 gezeigt, wobei insgesamt drei Abschnitte 54, 55 vorgesehen sind. Konkret umfasst das Implantat 51 gemäß Fig. 7 zwei Randabschnitte 54 und einen Mittelabschnitt 55. Die Randabschnitte 54 weisen denselben, konstanten Maschenwinkel auf. Der Mittelabschnitt 55 umfasst ebenfalls einen konstanten Maschenwinkel, der sich jedoch von dem Maschenwinkel der Randabschnitte 54 unterscheidet.

In den Fig. 6 und 7 sind zusätzlich einzelne Maschen 61 des Geflechts 60 dargestellt, um den abschnittsweise konstanten Maschenwinkel zu veranschaulichen.

In Fig. 7 ist gut erkennbar, dass das Implantat 41 ein Geflecht 60 aufweist, das anhand patientenspezifischer Außenkonturvorgaben hergestellt ist. Dabei ist in einem Mittelabschnitt 55, der unterschiedliche Querschnittsdurchmesser aufweist, ein konstanter, einheitlicher Maschenwinkel eingehalten. Ferner weisen die Randabschnitte 54, die ebenfalls in sich unterschiedliche Querschnittsdurchmesser aufweisen, einen konstanten, einheitlichen Maschenwinkel auf. Der Maschenwinkel zwischen den Randabschnitten 54 und dem Mittelabschnitt 55 unterscheidet sich. Insbesondere ist im Mittelabschnitt 55 ein kleinerer Maschenwinkel vorgesehen, als in den Randabschnitten 54.

Im Allgemeinen ist der Maschenwinkel innerhalb eines Abschnitts 54, 55 vorzugsweise konstant. Dies gilt sowohl in Längsrichtung des Abschnitts 54, 55, als auch in Umfangsrichtung des Abschnitts 54, 55, insbesondere in Umfangsrichtung des Geflechts 60.

Für alle Ausführungsbeispiele des endovaskulären Implantats 41 gilt, das die Drähte 43 vorzugsweise einen Drahtdurchmesser von 20 - 80 µm, insbesondere von 40 - 55 µm, aufweisen. Der Maschenwinkel beträgt in einzelnen Abschnitten 54, 55 des Geflechts 60 vorzugsweise zwischen 55° und 80°, insbesondere zwischen 65° und 75°. Insgesamt kann das Geflecht 60 aus mehreren Drähten 43 geflochten sein, wobei eine Drahtanzahl zwischen 12 und 96 Drähten, insbesondere zwischen 20 und 64 Drähten, bevorzugt ist. Die Drähte 43 weisen vorzugsweise ein Formgedächtnismaterial, insbesondere eine Nickel-TitanLegierung, vorzugsweise Nitinol, auf bzw. bestehen daraus.

Es ist im Allgemeinen möglich, Drähte 43 mit unterschiedlichen Drahtdurchmessern zu kombinieren. So können beispielsweise vereinzelt Drähte 43 in das Geflecht 60 integriert sein, die einen größeren Drahtdurchmesser als die übrigen Drähte des Geflechts 60 aufweisen. Derartige Drähte können eine stabilisierende Funktion für das Geflecht 60 übernehmen. Es ist auch möglich, Drähte 43 aus unterschiedlichen Materialien zu kombinieren, um das Geflecht 60 zu bilden. Beispielsweise können Drähte 43 aus Nitinol und Drähte 43 aus Polymer gemeinsam das Geflecht 60 bilden. Das Geflecht 60 kann beispielsweise durch eine Kombination von Nitinoldrähten mit Polymerfasern gebildet sein.

### Bezugszeichen

- 10: Erster Verfahrensschritt
- 11: Gefäßmodell
- 20: Zweiter Verfahrensschritt
- 21: Werkzeugmodell
- 22: Axiales Ende
- 30: Dritter Verfahrensschritt
- 31: Flechtwerkzeug
- 40: Vierter Verfahrensschritt
- 41: Implantat
- 42: Umfangswandung
- 43: Draht
- 44: Aneurysmenausbuchtung
- 50: Aneurysmenprojektion
- 51: Randbereich
- 52: Mittelbereich
- 53: Übergangsbereich
- 54: Randabschnitt
- 55: Mittelabschnitt
- 60: Geflecht
- 61: Masche

## Patentansprüche

1. Verfahren zum Herstellen eines patientenspezifischen, endovaskulären Implantats (41), beinhaltend die Erstellung eines Flechtwerkzeugs (31) und die Bildung des Implantats (41) durch Flechten von Drähten (43) um das Flechtwerkzeug (31),
**dadurch gekennzeichnet, dass**
das Verfahren die folgenden Schritte umfasst:
a. Bereitstellen eines dreidimensionalen Gefäßmodells (11) eines Blutgefäßes;
b. Bildung einer Durchmessersequenz entlang eines Zielabschnitts des Gefäßmodells (11), wobei in regelmäßigen Abständen Durchmesserwerte des Zielabschnitts ermittelt werden;
c. Bildung eines Werkzeugmodells (21) durch Übertragung der Durchmessersequenz auf eine lineare Achse;
d. Erstellung des Flechtwerkzeugs (31) in der Form des Werkzeugmodells (21); und
e. Bildung des Implantats (41) durch Flechten von Drähten (43) um das Flechtwerkzeug (31).

2. Verfahren nach Anspruch 1,
**dadurch gekennzeichnet, dass**
das Gefäßmodel (11) Bilderfassungsdaten aufweist, die durch ein angiographisches Bilderfassungssystem bereitgestellt werden.

3. Verfahren nach Anspruch 1 oder 2,
**dadurch gekennzeichnet, dass**
bei der Bildung des Werkzeugmodells (21) dessen Außenkontur geglättet und/oder dessen axiale Enden (22) modifiziert werden.

4. Verfahren nach einem der vorhergehenden Ansprüche,
**dadurch gekennzeichnet, dass**
die Schritte des Bereitstellens des Gefäßmodells (11), der Bildung der Durchmessersequenz und der Bildung des Werkzeugmodells (21) durch ein Bildbearbeitungsprogramm auf einem Rechner ausgeführt werden.

5. Verfahren nach einem der vorhergehenden Ansprüche,
**dadurch gekennzeichnet, dass**
beim Flechten der Drähte (43) eine Umfangswandung (42) mit einer Außenkontur gebildet wird, die der Innenkontur des Zielabschnitts entspricht.

6. Verfahren nach einem der vorhergehenden Ansprüche,
**dadurch gekennzeichnet, dass**
das endovaskuläre Implantat (41) radial komprimierbar und/oder expandierbar ist.

7. Verfahren nach einem der vorhergehenden Ansprüche,
**dadurch gekennzeichnet, dass**
das endovaskuläre Implantat (41) ein Stent ist.

8. Verfahren nach einem der vorhergehenden Ansprüche,
**dadurch gekennzeichnet, dass**
beim Flechten der Drähte (43) ein Flechtwinkel zwischen zwei sich kreuzenden Drähten (43) variiert wird derart, dass in Längsrichtung des Implantats (41) zumindest abschnittsweise ein konstanter Maschenwinkel gebildet wird.

9. Verfahren nach einem der vorhergehenden Ansprüche,
**dadurch gekennzeichnet, dass**
beim Flechten der Drähte (43) eine Abzugsgeschwindigkeit der Drähte (43) variiert wird derart, dass in Längsrichtung des Implantats (41) zumindest abschnittsweise ein konstanter Maschenwinkel gebildet wird.

10. Verfahren nach Anspruch 8 oder 9,
**dadurch gekennzeichnet, dass**
der konstante Maschenwinkel vollständig über einen Abschnitt des Implantats gebildet wird, der unterschiedliche Querschnittsdurchmesser aufweist

## Claims

1. A method for producing a patient-specific endovascular implant (41), including creating a braiding tool (31) and forming the implant (41) by braiding wires (43) around the braiding tool (31),
**characterised in that**
the method comprises the following steps:
a. providing a three-dimensional vessel model (11) of a blood vessel;
b. forming a diameter sequence along a target section of the vessel model (11), wherein diameter values of the target section are determined at regular space intervals;
c. forming a tool model (21) by transferring the diameter sequence to a linear axis;
d. creating the braiding tool (31) in the shape of the tool model (21); and
e. forming the implant (41) by braiding wires (43) around the braiding tool (31).

2. The method according to claim 1,
**characterised in that**
the vessel model (11) includes image capture data which are provided by an angiographic image-capturing system.

3. The method according to claim 1 or 2,
**characterised in that**
during formation of the tool model (21), the outer contour thereof is smoothed and/or the axial ends (22) thereof are modified.

4. The method according to any one of the preceding claims,
**characterised in that**
the steps of providing the vessel model (11), forming the diameter sequence and forming the tool model (21) are executed by an image-processing program on a computer.

5. The method according to any one of the preceding claims,
**characterised in that**
during braiding of the wires (43), a circumferential wall (42) is formed with an outer contour which corresponds to the inner contour of the target section.

6. The method according to any one of the preceding claims,
**characterised in that**
the endovascular implant (41) can be compressed and/or expanded radially.

7. The method according to any one of the preceding claims,
**characterised in that**
the endovascular implant (41) is a stent.

8. The method according to any one of the preceding claims,
**characterised in that**
during braiding of the wires (43), a braiding angle between two intersecting wires (43) is varied such that a constant mesh angle is formed, at least in some sections, in the longitudinal direction of the implant (41) .

9. The method according to any one of the preceding claims,
**characterised in that**
during braiding of the wires (43), a take-off speed of the wires (43) is varied such that a constant mesh angle is formed, at least in some sections, in the longitudinal direction of the implant (41).

10. The method according to claim 8 or 9,
**characterised in that**
the constant mesh angle is formed fully over a section of the implant which has different cross-sectional diameters.

## Revendications

1. Procédé destiné à fabriquer un implant endovasculaire (41) spécifique à un patient, comprenant la réalisation d'un outil de tissage (31) et la création de l'implant (41) par tissage de fils métalliques (43) autour de l'outil de tissage (31),
**caractérisé en ce que**
le procédé comporte les étapes suivantes :
a. de la mise à disposition d'un modèle de vaisseau (11) tridimensionnel d'un vaisseau sanguin ;
b. de la création d'une séquence de diamètres le long d'un tronçon cible du modèle de vaisseau (11), des valeurs diamétrales du tronçon cible étant déterminées à des écarts réguliers ;
c. de la création d'un modèle d'outil (21) par transfert de la séquence de diamètres sur un axe linéaire ;
d. de la réalisation de l'outil de tissage (31) sous la forme du modèle d'outil (21) ; et
e. de la création de l'implant (41) par tissage de fils métalliques (43) autour de l'outil de tissage (31).

2. Procédé selon la revendication 1,
**caractérisé en ce que**
le modèle de vaisseau (11) comporte des données de capture d'image qui sont mises à disposition par un système de capture d'images angiographiques.

3. Procédé selon la revendication 1 ou 2,
**caractérisé en ce que**
lors de la réalisation du modèle d'outil (21), on lisse son contour extérieur et/ou on modifie ses extrémités axiales (22).

4. Procédé selon l'une quelconque des revendications précédentes,
**caractérisé en ce que**
les étapes de la mise à disposition du modèle de récipient (11), de la création de la séquence de diamètres et de la création du modèle d'outil (21) sont réalisées par un programme de traitement d'images sur un ordinateur.

5. Procédé selon l'une quelconque des revendications précédentes,
**caractérisé en ce que**
lors du tissage des fils métalliques (43), on réalise une paroi circonférentielle (42) avec un contour extérieur qui correspond au contour intérieur du tronçon cible.

6. Procédé selon l'une quelconque des revendications précédentes,
**caractérisé en ce que**
l'implant (41) endovasculaire est radialement compressible et/ou expansible.

7. Procédé selon l'une quelconque des revendications précédentes,
**caractérisé en ce que**
l'implant (41) endovasculaire est un stent.

8. Procédé selon l'une quelconque des revendications précédentes,
**caractérisé en ce que**
lors du tissage des fils métalliques (43), on fait varier un angle de tissage entre deux fils métalliques (43) qui se croisent, de sorte que dans la direction longitudinale de l'implant (41), un angle de maillage constant soit réalisé au moins par tronçons.

9. Procédé selon l'une quelconque des revendications précédentes,
**caractérisé en ce que**
lors du tissage des fils métalliques (43), on fait varier une vitesse de dévêtissement des fils métalliques (43) de sorte que dans la direction longitudinale de l'implant (41), un angle de maillage constant soit réalisé au moins par tronçons.

10. Procédé selon la revendication 8 ou 9,
**caractérisé en ce**
**qu'**on créé l'angle de maillage constant totalement sur un tronçon de l'implant qui comporte des diamètres transversaux différents.
